# EUROPEAN PATENT APPLICATION

(11) **EP 4 592 392 A1**
(43) Date of publication of application: **30.07.2025**
(21) Application number: 23867070.7
(22) Date of filing: 30.06.2023
(51) Int. Cl.: C12N 15/12, C12Q 1/6886, C12Q 1/6869, C12Q 1/6851, C12N 15/11

(54) **NOVEL TUMOR DETECTION MARKER TAGME AND USE THEREOF**

(30) Priority: 22.09.2022 CN 202211157754
(71) Applicant: Shanghai Epiprobe Biotechnology Co., Ltd., Shanghai 200233 (CN)
(72) Inventor: LI, Zhenyan, Shanghai 200233 (CN); DONG, Shihua, Shanghai 200233 (CN); MAO, Zhanrui, Shanghai 200233 (CN)
(74) Representative: IPLodge bv
(86) International application number: PCT/CN2023/104682
(87) International publication number: WO 2024/060775

(57) **Abstract**

Provided are a tumor detection marker TAGMe and use thereof. On the basis of analysis and research on a large number of clinical samples, novel DNA methylation tumor markers are obtained by separation. The markers are in a low methylation state in a normal tissue and in a high methylation state in a tumor sample, and show a significant difference in the methylation state in apatient with a tumor, and the difference is statistically significant. The markers have a more excellent effect when they are used in combination for detection. The tumor markers can be used as markers for diagnosis, screening, typing, detection, and prognosis of al clinical tumor, can also be used as novel molecules for clinical auxiliary diagnosis or prognosis of a tumor, or can be used for designing a diagnostic reagent and kit.

## Description

The present disclosure claims the benefit of priority to Patent Application CN 202211157754.9, named "Novel tumor detection marker TAGMe and use thereof" and filed September 22nd, 2022, with its entire content incorporated herein by reference.

### Field of the disclosure

The present disclosure belongs to the field of gene epigenetic modification, more specifically, the present disclosure relates to a novel tumor detection marker and use thereof.

### Background of the disclosure

Endometrial cancer (EC) is one of the common malignant tumors in the female reproductive tract, second only to cervical cancer in incidence. With the improvement of living conditions, the incidence of EC is increasing year by year. It is estimated that by 2030 in near future, the global economic and healthcare burden caused by EC will rise by 60%. Although most EC cases occur in postmenopausal women, recent data show a significant increase in EC incidence among women aged 40 or younger, ranging from 2% to 14%. For young women of reproductive age, early diagnosis of EC at stages without myometrial invasion or extrauterine spread provides opportunities to preserve the uterus and/or ovaries. Therefore, early diagnosis of EC is crucial, as it can reduce female mortality and offer young patients a fertility-sparing or endocrine function-preserving treatment option.

However, there is currently a lack of reliable, effective, and non-invasive screening and diagnostic tools for early-stage EC. In early endometrial cancer, most patients lack obvious positive clinical signs. Multiple studies indicate that while 90% of EC patients present with vaginal bleeding, only 5-10% of women with abnormal vaginal bleeding are ultimately diagnosed with EC or precancerous lesions. Besides, there is currently a lack of reliable, effective, and non-invasive screening and diagnostic tools in clinical. Existing traditional diagnostic methods comprise: (1) Ultrasound: Transvaginal ultrasound (TVS) is the primary and most widely used non-invasive screening tool for postmenopausal bleeding. However, its high false-positive rate limits its ability to reliably distinguish benign from malignant endometrial changes, and EC may occur even in women without endometrial thickening. (2) Imaging: Imaging including magnetic resonance (pelvic MRI), CT, and PET-CT can determine lesion size and location but lack sensitivity for early EC or precancerous lesions, primarily serving for staging and grading. (3) Cytology: As a non-invasive method, cytological method exhibits highly specificity and low sensitivity. The positive rate of vaginal exfoliated cytology examination is low. Besides, cancer cells exfoliated from the uterine cavity often undergo dissolution and degeneration, making it difficult to identify after staining. (4) Serum tumor markers: EC lacks specific diagnostic markers. Some patients may show abnormal levels of CA125, CA19-9, CA153 or HE4. These levels are highly-correlated with histological subtypes, myometrial invasion depth, extrauterine spread and other factors and partially-correlated with endometrial malignancy itself. Due to the absence of minimally invasive triage methods, all patients with abnormal vaginal bleeding must undergo invasive procedures-dilation and curettage and/or hysteroscopic biopsy, that is pathological examination of endometrial tissues to confirm the diagnosis of EC. Most EC develops through a multi-year progression from endometrial hyperplasia to atypical hyperplasia (precancerous lesions). During this prolonged progression, patients with abnormal bleeding often endure repeated invasive endometrial biopsies, resulting in physical, psychological and financial burdens. Consequently, there is an urgent need to develop novel methods for early cancer detection.

### Summary of the disclosure

The aim of the present disclosure is to provide novel tumor detection markers TAGMe-1 and TAGMe-2, and a combination thereof. The markers exhibit low methylation levels in normal tissues and hypermethylation levels in endometrial cancer (EC) samples and can be used for EC detection. Samples for detection comprise but are not limited to tissues, cervical exfoliated cells, endocervical curettage specimens, vaginal secretions, and so on.

In the first aspect, the present disclosure provides a use of an isolated nucleic acid or a nucleic acid converted therefrom in preparing reagents or kits for tumor detection (comprising screening, diagnosis, detection or prognostic evaluation); wherein, the nucleic acid is: (1) a nucleic acid with a nucleotide sequence shown in SEQ ID NO: 1 or SEQ ID NO: 5, or a nucleic acid combination thereof; or (2) a nucleic acid with a sequence complementary to the nucleic acid of (1), or a nucleic acid combination thereof; wherein, the nucleic acid converted from the isolated nucleic acid is a nucleic acid corresponding to (1) or (2), with unmodified cytosine converted to T or U and modified cytosine C at CpG sites unchanged.

In one or more embodiments, the sequence of SEQ ID NO: 1 or SEQ ID NO: 5 also comprises variant sequences or homologous sequences. Preferably, the variant sequences or homologous sequences have more than 80%, more than 85%, more than 90%, more than 92%, more than 95%, more than 96%, more than 98%, more than 99%, more than 99.5%, or more than 99.8% sequence identity to the sequence shown in SEQ ID NO: 1 or SEQ ID NO: 5. Correspondingly, polynucleotides converted from the variant sequences or homologous sequence (with unmodified cytosine converted to T or U and modified cytosine C at CpG sites unchanged) are also encompassed.

In one or more embodiments, the nucleic acid with a sequence complementary to the nucleic acid of (1) is a nucleic acid with a nucleotide sequence shown in SEQ ID NO: 2 or SEQ ID NO: 6; the present disclosure also comprises variant sequences or homologous sequences of SEQ ID NO: 2 or SEQ ID NO: 6 with more than 80%, more than 85%, more than 90%, more than 92%, more than 95%, more than 96%, more than 98%, more than 99%, more than 99.5%, or more than 99.8% sequence identity to the sequence shown in SEQ ID NO: 2 or SEQ ID NO: 6. Correspondingly, polynucleotides converted from the variant sequences or homologous sequence (with unmodified cytosine converted to T or U and modified cytosine C at CpG sites unchanged) are also encompassed.

In one or more embodiments, the tumor comprises: endometrial cancer, cervical cancer (cervical squamous cell carcinoma and cervical adenocarcinoma), pancreatic cancer, head and neck cancer, colon cancer, colorectal cancer, esophageal cancer, lung cancer (squamous cell lung cancer, lung adenocarcinoma), cholangiocarcinoma.

In one or more embodiments, the tumor detection is for tumors (e.g., endometrial cancer) or precancerous lesions (e.g., atypical endometrial hyperplasia) thereof.

In one or more embodiments, samples for tumor detection comprise (but are not limited to): blood samples, tissue samples (such as paraffin-embedded samples), cervical samples, uterine cavity samples, pleural effusion samples, alveolar lavage Fluid sample, ascites sample, ascites lavage sample, bile sample, stool sample, urine sample, saliva sample, cerebrospinal fluid sample, cell smear sample, cell sample; preferably, the samples comprise (but are not limited to): cervical scraping or brushing sample, cervical swab, uterine cavity tissue (scratching), cervical exfoliated cells, endocervical curettage specimens, uterine lavage fluid, vaginal secretions.

In another aspect, the present disclosure provides a method for detecting the methylation level of a sample to be tested, wherein the method comprises: extracting nucleic acids from the sample to be tested; and detecting the modification status of CpG sites in a target sequence or fragment thereof within the extracted nucleic acids, wherein the target sequence is a nucleic acid converted from a nucleotide sequence shown in SEQ ID NO: 1 or SEQ ID NO: 5, or a nucleic acid combination thereof, with unmodified cytosine converted to T or U and modified cytosine C at CpG sites unchanged, corresponding to the nucleotide sequence shown in SEQ ID NO: 1 or SEQ ID NO: 5.

In one or more embodiments, the method for detecting the modification status of the CpG sites in the target sequence of extracted nucleic acids comprises pyrosequencing, bisulfite conversion sequencing, methylation microarray, methylation-specific PCR, methylation-sensitive restriction enzyme digestion, qPCR, digital PCR, next-generation sequencing, third-generation sequencing, whole-genome methylation sequencing, DNA enrichment assay, simplified bisulfite sequencing, HPLC, MassArray, or combinations thereof.

In one or more embodiments, the method for detecting the modification status of the CpG sites in the target sequence of extracted nucleic acids comprises: (i) treating the extracted nucleic acids to convert unmodified cytosine to uracil.

In one or more embodiments, the modification comprises 5-methylation (5mC), 5-hydroxymethylation (5hmC), 5-formylmethylation (5-fC) or 5-carboxymethylation (5-caC).

In one or more embodiments, it further comprises: (i) subjecting the nucleic acids to bisulfite treatment as described in step (i); and (ii) analyzing the modification status of the target sequence in the nucleic acids treated in step (i).

In one or more embodiments, the nucleic acid is a combination of "SEQ ID NO: 1 or a reverse complementary sequence thereof" and "SEQ ID NO: 5 or a reverse complementary sequence thereof".

In one or more embodiments, the abnormal methylation refers to hypermethylation of C at CpG sites of the nucleic acid.

In one or more embodiments, other methylation detection methods and methylation detection methods newly developed in the future can also be applied in the present disclosure.

In one or more embodiments, the methylation method is not a diagnostic method, that is, it is not intended to directly obtain diagnostic results of a disease.

In one or more embodiments, the method for detecting methylation of a sample is an in vitro method.

In one or more embodiments, the methylation-sensitive restriction enzyme is a restriction enzyme that is sensitive to methylated bases in recognition sites; comprising but not limited to: one or a combination of HpaII, Acil, Bsu15I, Hin1I, Hin6I, HpyCH4IV, NarI, etc.

In another aspect, the present disclosure provides a method for preparing a reagent for tumor detection (comprising screening, diagnosis, detection or prognostic evaluation), wherein the method comprises: providing a nucleic acid with a nucleotide sequence shown in SEQ ID NO: 1 or SEQ ID NO: 5, wherein the full-length nucleic acid or a fragment thereof is used as a target sequence, and designing a detection reagent for specifically detecting modifications at CpG sites of the target sequence.

In one or more embodiments, the detection reagent comprises but is not limited to: primers, probes, chips or test strips.

In one or more embodiments, one or more reagents can be prepared for the full-length nucleic acid or fragments of SEQ ID NO: 1.

In one or more embodiments, one or more reagents can be prepared for the full-length nucleic acid or fragments of SEQ ID NO: 5.

In one or more embodiments, the detection reagent is integrated on a chip.

In another aspect, the present disclosure provides a reagent or reagent combination for specifically detecting modifications at CpG sites in a target sequence, wherein the target sequence is a nucleic acid converted from a nucleotide sequence shown in SEQ ID NO: 1 or SEQ ID NO: 5, or a reagent combination thereof.

In one or more embodiments, the reagent or reagent combination targets a gene sequence comprising the target sequence.

In one or more embodiments, the gene sequence comprises gene Panel or gene cluster.

In one or more embodiments, preferably, the reagent or reagent combination is selected from one or more of the following groups: primers with the sequences of SEQ ID NO: 9 and SEQ ID NO: 10; primers with the sequences of SEQ ID NO: 11 and SEQ ID NO: 12; primers with the sequences of SEQ ID NO: 13 and SEQ ID NO: 14; or primers with the sequences of SEQ ID NO: 15 and SEQ ID NO: 16.

In another aspect, the present disclosure provides a use of the reagent or reagent combination for preparing kits for tumor detection (comprising screening, diagnosis, detection or prognostic evaluation); preferably the tumor comprises: endometrial cancer, cervical cancer, pancreatic cancer, head and neck cancer, colon cancer, colorectal cancer, esophageal cancer, lung cancer.

In another aspect, the present disclosure provides a kit for tumor detection (comprising screening, diagnosis, detection or prognostic evaluation), wherein the kit comprises the reagent or reagent combination.

In one or more embodiments, the kit also comprises but are not limited to: DNA purification reagents, DNA extraction reagents, bisulfite, PCR amplification reagents.

In one or more embodiments, the kit also comprises instructions indicating procedures for the detection and criteria for result determination.

In another aspect, the present disclosure provides an isolated nucleic acid or nucleic acid converted therefrom, wherein the nucleic acid is: (1) a nucleic acid with a nucleotide sequence shown in SEQ ID NO: 1 or SEQ ID NO: 5, or a nucleic acid combination thereof; or (2) a nucleic acid with a sequence complementary to the nucleic acid of (1), or a nucleic acid combination thereof; wherein, the nucleic acid converted from the isolated nucleic acid is a nucleic acid corresponding to (1) or (2), with unmodified cytosine converted to T or U and modified cytosine C at CpG sites unchanged.

In one or more embodiments, the modified CpG sites comprise CpG sites with 5-formylmethylation, 5-hydroxymethylation, 5-methylation or 5-carboxymethylation.

Other aspects of the present disclosure will be apparent to those skilled in the art based on the disclosure herein.

### Description of drawings

Figure 1: Heatmap of candidate markers for endometrial cancer (EC) and precancerous lesions.
Figure 2A-D. Performance of TAGMe-1 and TAGMe-2 as markers in other cancer types in the TCGA database.
Figure 3A-D. Targeted NGS results of tissues in Example 3.
Figure 4A-D. Analysis results of TAGMe-1 in Example 4.
Figure 5A-D. Me-qPCR results of cervical cells in Example 5.
Figure 6A-D. Me-qPCR results of cervical cells in Example 6.
Figure 7. Sequence information of the TAGMe-1 methylated tumor marker.
Figure 8. Sequence information of the TAGMe-2 methylated tumor marker.

### DETAILED DESCRIPTION

The present disclosure reveals that DNA methylation alterations play a critical role in the development of endometrial cancer and serve as markers for early screening, auxiliary diagnosis, therapeutic efficacy evaluation, recurrence monitoring, etc. In the present disclosure, based on extensive analysis of clinical samples, novel DNA methylation markers (TAGMe-1 and TAGMe-2) were identified. The markers exhibit low methylation levels in normal tissues and hypermethylation levels in tumor samples, especially endometrial cancer and other tumor samples, with statistically significant differences. When the markers are used in combination for detection, their diagnostic performance is significantly enhanced. Thus, the tumor markers of the present disclosure are applicable for clinical tumor diagnosis, screening, subtyping, detection, prognosis. They can also be used as new molecules for clinical auxiliary diagnosis or prognosis of tumors or can be used to design diagnostic reagents and kits.

In the present disclosure, the term "sample" or "samples" comprises a substance suitable for detection of DNA methylation status obtained from any individual (preferably a human) or isolated tissues, cells or body fluid (such as plasma). For example, samples for tumor detection comprise (but are not limited to): blood samples, tissue samples (such as paraffin-embedded samples), cervical samples, uterine cavity samples, pleural effusion samples, alveolar lavage Fluid sample, ascites sample, ascites lavage sample, bile sample, stool sample, urine sample, saliva sample, cerebrospinal fluid sample, cell smear sample, cell sample; preferably, the samples comprise (but are not limited to): cervical scraping or brushing sample, cervical swab, uterine cavity tissue (scratching), cervical exfoliated cells, endocervical curettage specimens, uterine lavage fluid, vaginal secretions.

In the present disclosure, the term "hypermethylation" refers to high levels of CpG methylation, hydroxymethylation, formylmethylation or carboxymethylation within a gene sequence. For example, in terms of methylation-specific PCR (MSP) analysis, a positive PCR result obtained by a PCR reaction performed with methylation-specific primers can indicate that the tested DNA (gene) region is in a high methylation state. For example, in terms of real-time quantitative methylation-specific PCR, hypermethylation can be determined based on statistical differences of relative values compared to the control sample.

In the present disclosure, the term "tumor" refers to a type of tumors defined by hypermethylation at SEQ ID NO: 1 or SEQ ID NO: 5 in the genome as described in the present disclosure.

As used in the present disclosure, the "detection" comprises screening, diagnosis, detection or prognostic assessment.

As used in the present disclosure, the term "endometrial cancer" broadly comprises "endometrial cancer and precancerous lesions thereof", wherein the latter is also called "endometrial atypical hyperplasia" or "early endometrial cancer". However, it can be understood that in certain circumstances, such as in some embodiments, when the "endometrial cancer" is classified with "endometrial cancer precancerous lesions", the term "endometrial cancer" refers to cancer that has passed the precancerous stage, for example at the advanced stage.

In the present disclosure, it is proposed that some meaningful epigenetic changes are important factors in the occurrence of endometrial cancer. Compared with DNA mutations, specific regions of the genome exhibit a hypermethylation state, wherein the hypermethylation state occurs earlier and more stably in early diagnosis of tumors. Abnormal DNA methylation patterns can be used to predict the risk of progression of atypical hyperplasia to endometrial cancer. The DNA hypermethylation state runs through the entire process of normal cells turning into tumor cells and occurs in the ultra-early stage of tumors, ahead of genetic mutations. Therefore, tumor detection based on DNA hypermethylation status can be applied to ultra-early tumor screening, auxiliary diagnosis, efficacy evaluation and recurrence monitoring, covering the entire process of tumor diagnosis and treatment.

On this basis, the present disclosure provides a set of methylation markers, TAGMe-1 and TAGMe-2, as molecular detection markers for endometrial cancer. The markers exhibit low methylation levels in normal tissues and hypermethylation levels in endometrial cancer and precancerous lesions (atypical hyperplasia) tissues. Non-invasive auxiliary diagnosis of early endometrial cancer and precancerous lesions can be achieved through exfoliated cells from the cervix and uterine cavity, filling the gap in existing endometrial cancer screening technology. Besides, through further development, relatively simple, cheap, reliable and high-throughput clinical testing can be achieved.

In the present disclosure, there is a significant difference in the methylation status of the nucleotide sequence shown in SEQ ID NO: 1 or SEQ ID NO: 5 or its partial region (fragment) between tumor tissue and non-tumor tissue. When there is abnormal hypermethylation detected in the gene sequence region, it can be considered that the subject suffers from tumors or belongs to a high-risk group for tumors. This significant difference in the methylation status of the gene sequence shown in SEQ ID NO: 1 or SEQ ID NO: 5 or its partial region is very significantly present in endometrial cancer (comprising early stages). At the same time, such significant differences in methylation status also exist in other tumors, comprising: cervical cancer (cervical squamous cell carcinoma and cervical adenocarcinoma), pancreatic cancer, head and neck cancer, colon cancer, colorectal cancer, esophageal cancer, lung cancer (squamous cell lung cancer and lung adenocarcinoma), cholangiocarcinoma, etc.

The present disclosure also comprises "conservative variant sequences" of SEQ ID NO: 1 (or a reverse complementary sequence thereof) or SEQ ID NO: 5 (or a reverse complementary sequence thereof) with high conservation or sequence identity to the sequence of SEQ ID NO: 1 (or its reverse complementary sequence) or SEQ ID NO: 5 (or its reverse complementary sequence). The "high sequence identity" is for example sequence identity of more than 90%, more than 92%, more than 95%, more than 98%, more than 99%, and so on. It can be understood that there may be differences in individual sequence positions between different biological individuals (for example, there may be some meaningless SNPs), but this does not affect the detection based on the overall scheme of the present disclosure.

According to the above, the present disclosure provides nucleic acids derived from specific regions of the human genome with the sequences shown in SEQ ID NO: 1 or SEQ ID NO: 5 (or fragments thereof), also comprising their antisense strands. In tumor cells, 5-methylcytosine (5mC) is formed at multiple 5'-CpG-3' cytosine positions within the nucleic acid sequences.

It is possible to detect one or more CpG provided by the present disclosure, so the present disclosure also comprises nucleic acid fragments of the nucleotide sequence, comprising at least one methylated CpG site. For SEQ ID NO: 1 (or a reverse complementary sequence thereof), the at least one methylated CpG sites may comprise 2 to 53 CpG sites (more specifically, such as 3, 5, 8, 10, 12, 15, 18, 20, 30, 40, 50). For SEQ ID NO: 5 (or a reverse complementary sequence thereof), the at least one methylated CpG sites may comprise 2 to 52 CpG sites (more specifically, such as 3, 5, 8, 10, 12, 15, 18, 20, 30, 40, 50). Tcan understandafter the present disclosure provides CpG numbering based on one DNA , the numbering of each CpG site in the complementary DNA corresponding to the sense strand can be easily obtained based on the content provided by the present disclosure

Based on the genomic fragment information provided by the present disclosure, those skilled in the art can readily identify and utilize these CpG sites. The examples of the present disclosure provide a series of sequence fragments comprising CpG sites. These fragments can be used as some examples of preferred embodiments. However, it can be understood that based on the information provided by the present disclosure, modifications, such as selecting longer sequences comprising the disclosed sequences or overlapping regions with the sequences of the present disclosure, are permissible.

The present disclosure also comprises gene Panel or gene cluster comprising the nucleotide sequence shown in SEQ ID NO: 1 or SEQ ID NO: 5, sequence fragments or complementary sequences thereof (SEQ ID NO: 2 or SEQ ID NO: 6). The gene Panel or gene cluster can also be used to obtain features of normal cells and tumor cells through DNA methylation state detection.

It can be understood that a variety of methods for analyzing the methylation state can be applied in the present disclosure, and there are no specific limitations on such detection methods. The nucleic acids provided by the present disclosure can be used as key regions in the genome to analyze methylation states and their methylation states can be analyzed using various methods known in the art, thereby enabling analysis of tumor occurrence or development.

The nucleic acids described in SEQ ID NO: 1 or SEQ ID NO: 5, fragments or complementary sequences thereof, can be treated with bisulfite to convert unmethylated cytosine into uracil and methylated cytosine unchanged. Therefore, the present disclosure further provides nucleic acids obtained after bisulfite treatment of the aforementioned nucleic acids (comprising a complementary strand (an antisense strand thereof)), comprising: nucleic acids or nucleotide fragments with the nucleotide sequence shown in SEQ ID NO: 3 or SEQ ID NO: 7, or nucleic acids or nucleotide fragments with the nucleotide sequence shown in SEQ ID NO: 4 or SEQ ID NO: 8. These nucleic acids can be used as more direct targets for designing detection reagents or detection kits.

The nucleic acids of the nucleotide sequence shown in SEQ ID NO: 1 or SEQ ID NO: 5, and/or their complementary nucleic acids, and/or one or more of their fragments, may be incorporated into one or several entities, such as one or more nucleic acid sets, for use by those skilled in the art. For example, one or more nucleic acids or nucleic acid fragments can be selected from this nucleic acid set to design target analysis reagents. The designed target analysis reagents may also be integrated into one or more entities, such as one or more reagent kits.

The nucleic acids derived from the nucleotide sequence shown in SEQ ID NO: 1 or SEQ ID NO: 5, and/or their complementary nucleic acids, and/or one or more of their fragments, after conversion (such as bisulfite conversion), may also be incorporated into one or several entities, such as one or more nucleic acid sets, for use by those skilled in the art. For example, one or more nucleic acids or nucleic acid fragments can be selected from this nucleic acid set to design target analysis reagents. The designed target analysis reagents may also be integrated into one or more entities, such as one or more kits or one or more chips.

As a preferred embodiment of the present disclosure, nucleic acids and/or their complementary nucleic acids and/or one or more fragments derived from the nucleotide sequence shown in SEQ ID NO: 1, can be used for detection in combination with nucleic acids and/or their complementary nucleic acids and/or one or more fragments derived from the nucleotide sequence shown in SEQ ID NO: 5.

Based on the target genes and their epigenetic features provided by the present disclosure, well-known methods in the art, as well as methods under development, can be applied in the present disclosure to implement methylation level detection. The methylation of nucleic acids can be determined using existing methods (such as methylation-specific PCR (MSP) or real-time quantitative methylation-specific PCR (Methylight), or other developing or future methods. For example, when detecting methylation levels, quantitative methylation-specific PCR (QMSP) methods can be used and this method is based on optical monitoring during fluorescence PCR, making them more sensitive than MSP. This method has high throughput and avoids the need for electrophoresis to analyze results. Other available methods comprise: qPCR (Me-qPCR), next-generation sequencing, pyrosequencing, Sanger sequencing, bisulfite conversion sequencing, whole-genome methylation sequencing, DNA enrichment assay, simplified bisulfite sequencing, HPLC, and combined gene group detection methods, and other standard methods in the field. Although some preferred embodiments are provided in the present disclosure, the overall scheme is not limited to these.

As a preferred embodiment of the present disclosure, a method for detecting the methylation of nucleic acids in a sample in vitro is also provided. The principle of the method is as follows: bisulfite can convert unmethylated cytosines to uracil, wherein the uracil will convert into thymine during subsequent PCR amplification, while methylated cytosines remain unchanged. Therefore, after bisulfite treatment, methylation sites generate a C/T nucleotide polymorphism (SNP). Based on this principle, the methylation of nucleic acids in the sample can be effectively distinguished between methylated and unmethylated cytosines.

The method of the present disclosure comprises: first, providing a sample and extracting genomic DNA; second, utilizing bisulfite treatment of the genomic DNA such that unmethylated cytosines are converted to uracil; and third, analyzing whether an abnormal methylation exists in the genomic DNA after treatment of step (b).

The method of the present disclosure can be used for: detecting a subject's sample to evaluate whether the subject has a tumor, or for distinguishing a high-risk tumor population. The method can also be used in cases where direct disease diagnosis is not the primary goal, such as in population regional analysis studies, scientific research, census, etc.

In a preferred embodiment of the present disclosure, DNA methylation is detected by PCR amplification and pyrosequencing. However, this method is not limited to this; other known or improving DNA methylation detection methods can also be applied. In PCR amplification, the primers used are not limited to those provided in the embodiment; primers with sequence differences from those provided in the embodiment but still targeting the nucleic acids or corresponding CpG sites indicated in the present disclosure may also be obtained.

As a preferred embodiment of the present disclosure, a method for detecting the methylation status of nucleic acids in a sample in vitro is also provided, wherein the method is based on methylation-sensitive restriction endonuclease digestion (MSRE). When a methylated base is present at the cleaved site, the methylation-sensitive restriction enzyme cannot cleave the DNA. The MSRE method is based on the principle that methylation-sensitive type II restriction enzymes cannot cleave DNA comprising one or more methylated recognition sequences. The fragments comprising one or more methylated CpG sequences can be cleaved by methylation-sensitive type II restriction enzymes or isoenzymes thereof and then analyzed using DNA blotting. The advantages of this method comprise: not requiring detailed primary structure of the target DNA and providing a direct evaluation of the methylation status of CpG islands, comprising obtaining quantitative analysis information on methylation of the tested genes.

Regarding the marker nucleic acids provided by the present disclosure, other methods and reagents known to those skilled in the art for determining genomic sequences, variants and methylation states thereof can also be included in the present disclosure.

The present disclosure provides a method for preparing tumor detection reagents, comprising: providing the nucleic acids as the target sequences, using the full length or fragment of the nucleic acid as a target sequence, designing specific detection reagents to detect the target sequences; wherein the target sequences comprise at least one methylated CpG site. The detection reagents may comprise but are not limited to: chips, primers, probes, etc. Once the markers are obtained, the selection of detection reagents can be done by those skilled in the art.

Once the nucleic acid sequence is known, primer design is a process well known to those skilled in the art. Two primers are placed on either side of the specific sequence of the target gene to be amplified (including CpG sequences; those complementary to the CpG in the gene region targeted for methylation and those complementary to the TpG in the gene region targeted for demethylation). After knowing the sequence of the nucleic acid, it is known to those skilled in the art to design primers. The two primers are on both sides of the specific sequence of the target gene to be amplified (comprising CpG sequence, wherein gene regions complementary to the CpG are targeted for original methylation gene regions and gene regions complementary to the TpG are targeted for original demethylation gene regions). In the preferred example of the present disclosure, the reagent is a primer, and the primers are preferably those listed in Tables 2 and 6. In addition to primers, other diagnostic or detection reagents can also be prepared, comprising but not limited to probes, chips, etc.

The reagent may also be a reagent combination, such as a primer combination. For example, the combination comprises more than one set of primers, so that multiple nucleic acids can be separately amplified.

The present disclosure also provides a kit for detecting the methylation of nucleic acids in a sample in vitro. The kit comprises: a container, and the above primers placed in the container.

The kit may also comprise various reagents required for DNA extraction, DNA purification, PCR amplification, etc., as well as other reagents, such as sample processing reagents. Additionally, the kit may comprise an instruction for use, wherein the instruction specifies the steps for detection and criteria for result determination for the convenience of use by those skilled in the art.

When the method and reagents of the present disclosure are used for diagnosing clinical tumors, their accuracy is very high, as demonstrated in the detection of various clinical tumor samples in the present disclosure. The present disclosure can be applied for tumor screening, efficacy evaluation, auxiliary diagnosis, prognosis monitoring and other fields, or in situations where direct disease diagnosis is not the goal, as mentioned earlier.

The tumor markers provided by the present disclosure, such as those for endometrial cancer, have very high specificity and sensitivity when used alone. When two markers are used in combination, their sensitivity and specificity are further improved. Therefore, the markers of the present disclosure have great application value in tumor auxiliary diagnosis, efficacy evaluation, prognosis monitoring, and other fields.

The tumor markers provided by the present disclosure, such as those for endometrial cancer, can be used for detecting cervical exfoliated cells, with easy and non-invasive sample collection. Compared to the need for surgical tissue sampling in clinical, the discomfort to the subject is greatly reduced, compliance is better, and the operation for clinicians is easier. Clearly, this represents a significant advancement.

The disclosure is further described below in conjunction with specific embodiments. It can be understood that these examples are merely for illustrative purposes rather than intended to limit the scope of the disclosure. The experimental methods without specifying the specific conditions in the following examples generally used the conventional conditions, such as those described in J. Sambrook, Molecular Cloning: A Laboratory Manual (3rd ed. Science Press) or followed the manufacturer's recommendation.

### Example 1. Screening of endometrial cancer detection markers

A total of 43 clinical frozen tissue samples were collected from five groups comprising normal endometrium, endometrial hyperplasia, atypical endometrial hyperplasia (precancerous lesions), endometrial cancer and paired paracancerous tissues. The methylation data at the whole genome level were analyzed using reduced representation bisulfite sequencing (RRBS) to screen for DNA methylation markers specific for endometrial cancer and precancerous lesions.

### 1. Acquisition of clinical samples

A total of 43 frozen tissue samples, each approximately the size of a soybean, were collected from five groups: normal endometrium, endometrial hyperplasia, atypical endometrial hyperplasia (precancerous lesions), endometrial cancer and paired paracancerous tissues.

### 2. DNA extraction

The Epiprobe Biotech Genomic DNA Extraction Kit (Epiprobe Biotech, K-21) was used for genomic DNA extraction from the samples (but the nucleic acid extraction method of the present disclosure is not limited to this method).

### 3. RRBS library construction

Extracted sample gDNA was digested with the MspI restriction enzyme, followed by magnetic bead-based size selection of suitably sized fragments and subsequent steps comprising end-repair, dA-tailing and adapter ligation were completed.

### 4. Bisulfite treatment

The above library samples were modified using bisulfite. In this experiment, the EZ DNA Methylation-Gold Kit (catalog no. D5006) from ZYMO Research was used, strictly following the steps in the instruction manual (but the present disclosure is not limited to this kit).

### 5. PCR amplification and product purification

Each library sample was amplified using primers with different molecular tags and the amplification products were selected and purified using magnetic beads. The concentration was determined prior to sequencing.

### 6. RRBS sequencing

Illumina Hiseq 2000 was used to perform 2×150 bp sequencing on the genomic RRBS libraries of the tissue samples. The sequencing data were subjected to quality control, alignment and other steps, yielding basic information such as the effective number of sequencing reads, the number of CpG sites measured and the coverage depth for each sample, as shown in Table 1. The total data amount of the RRBS library reached 198.31 G, with the normal endometrium group (Group A, NE), endometrial hyperplasia group (Group B, EH), atypical hyperplasia group (Group C, AH), endometrial cancer group (Group D, EC) and paracancerous tissue group (Group E, PC) obtaining an average of 16,686,194 M, 16,847,536 M, 14,942,502 M, 14,729,170 M, and 14,637,777 M sequencing reads, respectively. The number of all CpG sites with a coverage depth of five or more was 4,055,958 M, 4,169,395 M, 4,369,673 M, 3,984,048 M, and 4,271,290 M, respectively. The statistical data and preliminary experimental analysis indicate that the overall quality of the RRBS sequencing was good, the sequencing depth and CpG coverage were excellent, the data distribution among the groups was uniform and the results are highly reliable.

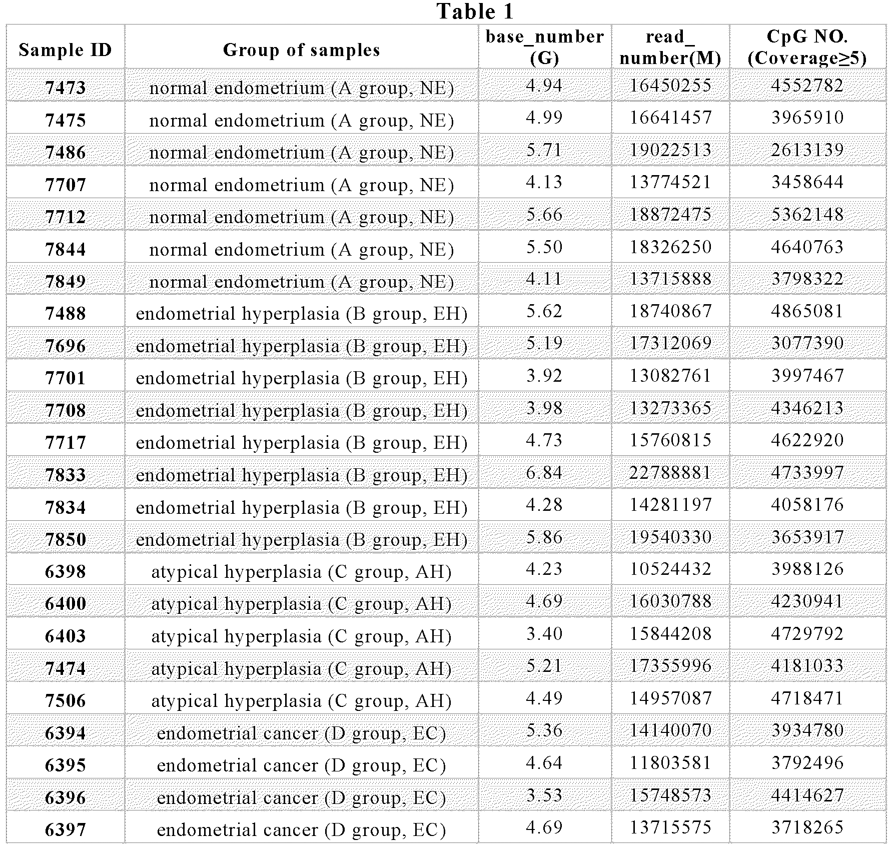

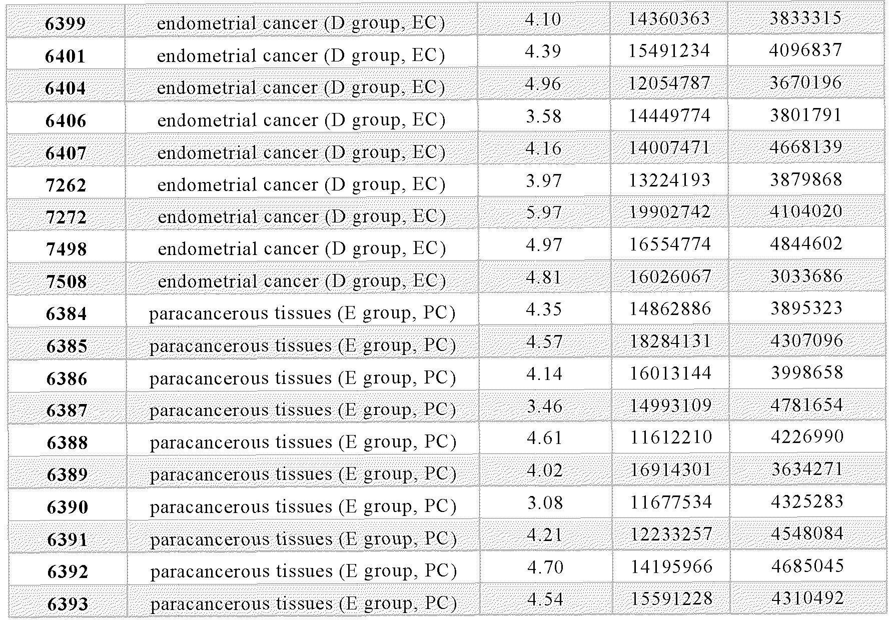

### 7. Marker screening

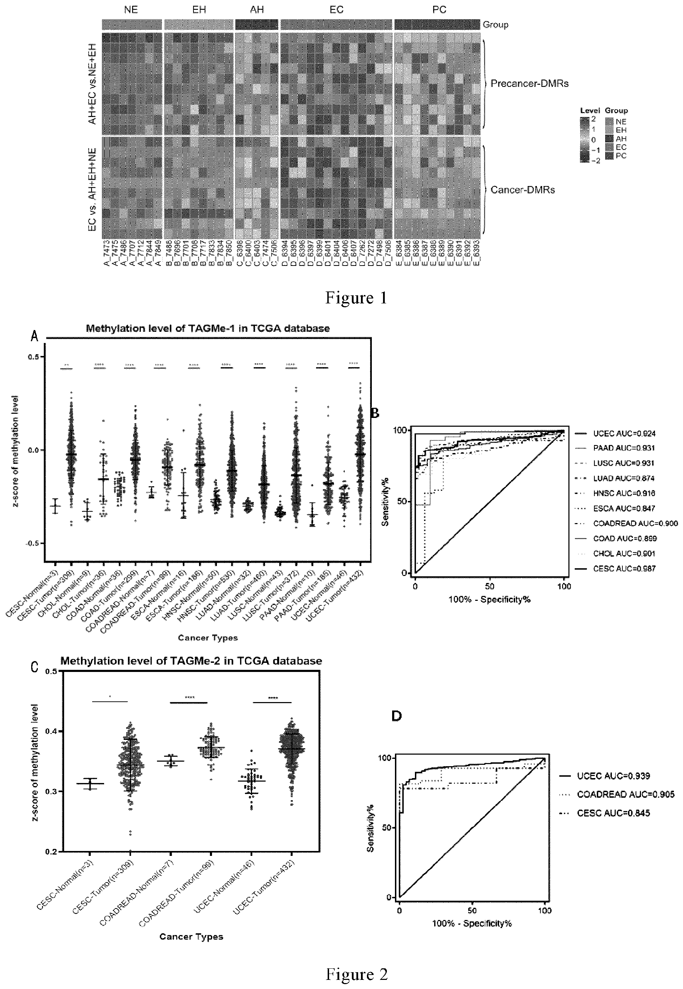

By analyzing methylation sequencing data among different stages and combining experimental analysis, specific methylation markers present in atypical endometrial hyperplasia (precancerous lesions) and/or cancer stages were screened. As shown in Figure 1, a heat map of a small subset of candidate diagnostic markers for endometrial cancer and precancerous lesions is provided. The inventors of the present disclosure obtained two groups of candidate methylation markers for endometrial cancer detection, namely, Precancer-DMRs for precancerous lesions and Cancer-DMRs for endometrial cancer, corresponding to DMRs that exhibit significantly high methylation only in the AH and EC stages or only in the EC stage and low methylation in other stages including precancerous tissues.

### (1) TAGMe-1

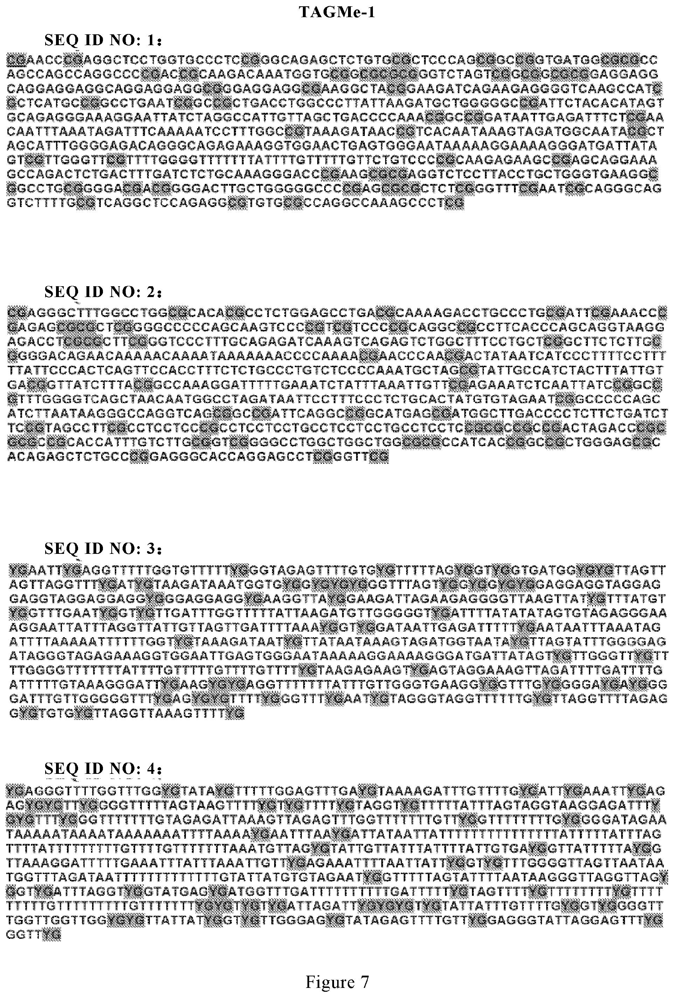

The nucleotide sequence of the obtained TAGMe-1 methylation tumor marker is as shown in SEQ ID NO: 1, as depicted in Figure 7, wherein the background-colored sites indicate potential methylated CG sites (53 groups).

The reverse complementary sequence of SEQ ID NO: 1 is as shown in SEQ ID NO: 2, as depicted in Figure 7, wherein the background-colored sites indicate potential methylated CG sites.

The sequence of SEQ ID NO: 1 after bisulfite conversion is as shown in SEQ ID NO: 3, as depicted in Figure 7, wherein the background-colored sites indicate potential methylated CG sites and Y represents C or T.The reverse complementary sequence of SEQ ID NO: 3 after bisulfite conversion is as shown in SEQ ID NO: 4, as depicted in Figure 7, wherein the background-colored sites indicate potential methylated CG sites and Y represents C or T.

### (2) TAGMe-2

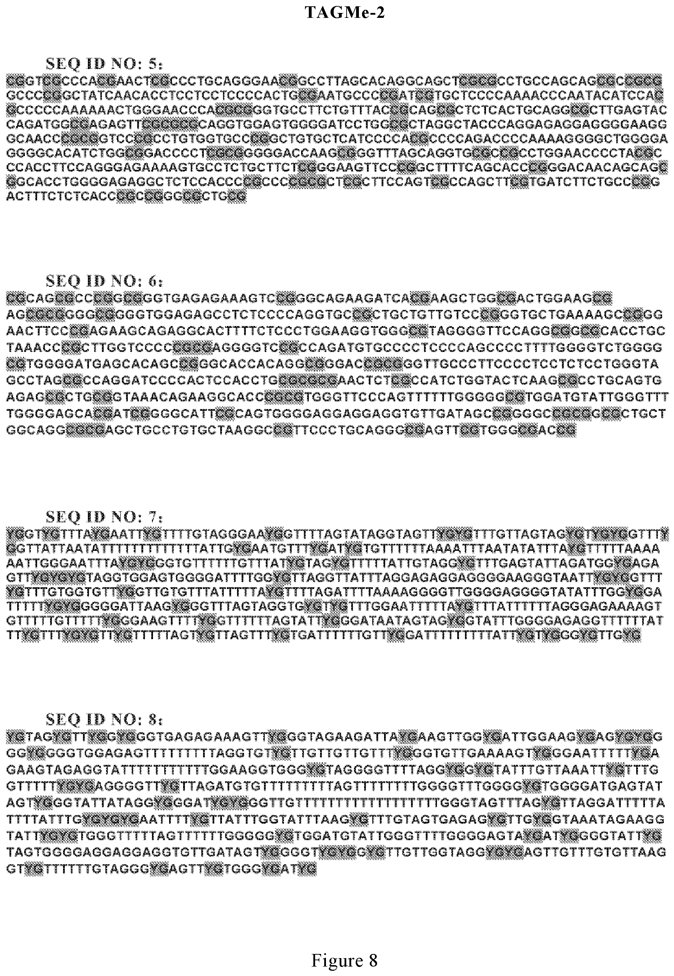

The nucleotide sequence of the obtained TAGMe-2 methylation tumor marker is as shown in SEQ ID NO: 5, as depicted in Figure 8, wherein the background-colored sites indicate potential methylated CG sites (52 groups).

The reverse complementary sequence of SEQ ID NO: 5 is as shown in SEQ ID NO: 6, wherein the background-colored sites indicate potential methylated CG sites.

The sequence of SEQ ID NO: 1 after bisulfite conversion is as shown in SEQ ID NO: 7, as depicted in Figure 8, wherein the background-colored sites indicate potential methylated CG sites and Y represents C or T.

The reverse complementary sequence of SEQ ID NO: 7 after bisulfite conversion is as shown in SEQ ID NO: 8, as depicted in Figure 8, wherein the background-colored sites indicate potential methylated CG sites and Y represents C or T.

### 8. Verification of the clinical performance of candidate DNA methylation markers using pyrosequencing

To further verify whether the candidate markers screened above have clinical application value, the markers of the present disclosure were compared with other Precancer-DMRs that were also analyzed to have methylation differences and significance (the top-ranking Precancer-DMRs in the heat map of Figure 1), using pyrosequencing technology in another set of collected clinical tissue samples. A total of 45 clinical tissue samples were collected, comprising tissues from 14 subjects with normal endometrium (NE), 11 subjects with endometrial hyperplasia (without atypical hyperplasia, EH), 5 subjects with atypical hyperplasia (AH) and 15 subjects with endometrial cancer (EC).

The primers for pyrosequencing amplification are as shown in Table 2.

**Table 2**

| **Target** | **Primer** | **Sequences (5'-3')** |
|---|---|---|
| **TAGMe-1** | Forward primer | gaggaggtaggaggaggtaggaggagg (SEQ ID NO: 9) |
| | Reverse primer | Biotin-aattcctttccctctacactatatataaaat (SEQ ID NO: 10) |
| **TAGMe-2** | Forward primer | tttaaaaggggttggggaggggtatattt (SEQ ID NO: 11) |
| | Reverse primer | Biotin-tctccccaaataccrctactattatccc (SEQ ID NO: 12) |

The performance indicators of candidate methylation markers in clinical tissue samples for diagnosing endometrial precancerous lesions or higher-grade are shown in Table 3.

**Table 3**

| **Target** | **Area Under the Curve of ROC (AUC)** | **Standard deviation** | **95% CI** | **P value** |
|---|---|---|---|---|
| **TAGMe-1** | **0.938** | 0.032 | 0.874-1.000 | **<0.0001** |
| **TAGMe-2** | **0.925** | 0.029 | 0.886-0.955 | **<0.0001** |
| **Chr5P15.33** | 0.776 | 0.076 | 0.628 to 0.924 | **0.0016** |
| **Chr12Q24.22** | 0.720 | 0.079 | 0.566-0.874 | **0.012** |
| **C11JH72** | 0.610 | 0.087 | 0.439-0.780 | 0.209 |

According to the above, the AUC of the two DMRs TAGMe-1 and TAGMe-2 are 0.938 and 0.925, respectively, both above 0.9. For early-stage (Precancer) patients with endometrial cancer, a very high AUC is achieved, which is unexpected. It demonstrates that their performance as methylation markers for early screening endometrial cancer is excellent.

### Example 2. Performance verification of TAGMe-1 and TAGMe-2

The TCGA database, as a recognized tumor database, comprises data from various tumors. For a type of tumor, it records a series of expression data, miRNA expression data, methylation data, mutation data and copy number data. Therefore, the present disclosure validated the performance of TAGMe-1 and TAGMe-2 using this database.

Download of multi-cancer methylation data from the TCGA database: All methylation 450K chip data of tumor samples in the TCGA database were downloaded, comprising 11,087 tumor and normal control samples. A total of 35 tumor types and sample sizes involved are shown in Table 4. ***total number of samples***

**Table 4. 35 tumor methylation data set information**

| **Name of database** | **Tumor type** | **Total number of samples** |
|---|---|---|
| ACC DNA methylation (Methylation450k) | Adrenocortical carcinoma | 80 |
| BLCA DNA methylation (Methylation450k) | Urothelial carcinoma of the bladder | 434 |
| BRCA DNA methylation (Methylation450k) | Invasive breast carcinoma | 872 |
| CESC DNA methylation (Methylation450k) | Cervical squamous cell carcinoma and cervical adenocarcinoma | 312 |
| CHOL DNA methylation (Methylation450k) | Cholangiocarcinoma | 45 |
| COAD DNA methylation (Methylation450k) | Colon cancer | 337 |
| COADREAD DNA methylation (Methylation450k) | Colorectal cancer | 443 |
| DLBC DNA methylation (Methylation450k) | Diffuse large B-cell lymphoma | 48 |
| ESCA DNA methylation (Methylation450k) | Esophageal carcinoma | 202 |
| GBM DNA methylation (Methylation450k) | Glioblastoma multiforme | 155 |
| HNSC DNA methylation (Methylation450k) | Head and neck cancer | 580 |
| KICH DNA methylation (Methylation450k) | Chromophobe renal cell carcinoma | 66 |
| KIRC DNA methylation (Methylation450k) | Clear cell renal cell carcinoma | 480 |
| KIRP DNA methylation (Methylation450k) | Papillary renal cell carcinoma | 321 |
| LAML DNA methylation (Methylation450k) | Acute myeloid leukemia | 194 |
| LGG DNA methylation (Methylation450k) | Low-grade glioma | 530 |
| LIHC DNA methylation (Methylation450k) | Liver cancer | 429 |
| LUAD DNA methylation (Methylation450k) | Lung adenocarcinoma | 492 |
| LUNG DNA methylation (Methylation450k) | Lung cancer | 907 |
| LUSC DNA methylation (Methylation450k) | Lung squamous cell carcinoma | 415 |
| MESO DNA methylation (Methylation450k) | Mesothelioma | 87 |
| OV DNA methylation (Methylation450k) | Ovarian serous cystadenocarcinoma | 10 |
| PAAD DNA methylation (Methylation450k) | Pancreatic cancer | 195 |
| PCPG DNA methylation (Methylation450k) | Pheochromocytoma and paraganglioma | 187 |
| PRAD DNA methylation (Methylation450k) | Prostate cancer | 549 |
| READ DNA methylation (Methylation450k) | Rectal adenocarcinoma | 106 |
| SARC DNA methylation (Methylation450k) | Sarcoma | 269 |
| SKCM DNA methylation (Methylation450k) | Cutaneous melanoma | 476 |
| STAD DNA methylation (Methylation450k) | Gastric adenocarcinoma | 398 |
| TGCT DNA methylation (Methylation450k) | Testicular germ cell tumor | 156 |
| THCA DNA methylation (Methylation450k) | Thyroid cancer | 571 |
| THYM DNA methylation (Methylation450k) | Thymoma | 126 |
| UCEC DNA methylation (Methylation450k) | Endometrial cancer | 478 |
| UCS DNA methylation (Methylation450k) | Uterine sarcoma | 57 |
| UVM DNA methylation (Methylation450k) | Uveal melanoma | 80 |

Calculation of the methylation values of TAGMe-1 and TAGMe-2: First, the average methylation values for TAGMe-1 and TAGMe-2 in different tumor tissue samples and control normal tissue samples were calculated, respectively, and then the difference between the average methylation values in the tumor samples and the control samples for each tumor type was calculated.

Analysis of the diagnostic performance of TAGMe-1 and TAGMe-2 in other cancers: The differences in the methylation values of TAGMe-1 and TAGMe-2 between tumor samples and control samples in various cancer types are shown in Figure 2A and Figure 2C. Through a two-sided Mann-Whitney t-test analysis, TAGMe-1 exhibits statistically significant differences in methylation levels between the tumor group and the control group in multiple tumor types, comprising endometrial cancer, cervical cancer, cholangiocarcinoma, colorectal cancer (colon cancer, rectal cancer), esophageal cancer, lung cancer (lung squamous cell carcinoma, lung adenocarcinoma), head and neck cancer, pancreatic cancer, etc. For TAGMe-2, there were statistically significant differences in methylation levels between the tumor group and the control group in colorectal cancer, endometrial cancer and cervical cancer respectively. The corresponding diagnostic AUC and performance are shown in Figures 2B, 2D and Table 5.

**Table 5. Performance table as markers in other cancer types**

| **TAGMe-1** | **Cutoff** | **Sensitivity%** | **Specificity%** |
|---|---|---|---|
| CESC | > -0.2644 | 97.73 | 100 |
| CHOL | > -0.2629 | 69.44 | 100 |
| COAD | > -0.1195 | 83.61 | 89.47 |
| COADREAD | > -0.1692 | 89.9 | 85.71 |
| ESCA | > -0.2056 | 87.1 | 81.25 |
| HNSC | > -0.2034 | 78.87 | 92 |
| LUAD | > -0.2681 | 78.65 | 96.88 |
| LUSC | > -0.3001 | 87.6 | 93.02 |
| PAAD | > -0.3147 | 92.93 | 90 |
| UCEC | > -0.1681 | 82.41 | 97.83 |
| | | | |
| **TAGMe-2** | **Cutoff** | **Sensitivity%** | **Specificity%** |
| UCEC | > 0.3366 | 89.81 | 89.13 |
| COADREAD | > 0.3611 | 81.82 | 100 |
| CESC | > 0.3206 | 78.32 | 100 |

Therefore, TAGMe-1 and TAGMe-2 are with diagnostic utility in many types of tumors.

### Example 3: DNA methylation detection in endometrial cancer-paracancerous tissue samples: target-NGS method

In this example, DNA methylation analysis in endometrial cancer-paracancerous tissue samples is performed using the target-NGS method. The steps are as follows:
1. Acquisition of clinical samples
20 cases of normal endometrial tissue/paracancerous tissue samples, 6 cases of endometrial atypical hyperplasia samples and 20 cases of endometrial cancer tissue samples were obtained clinically. The normal endometrial tissue/paracancerous tissue samples served as the control group, and the atypical hyperplasia and endometrial cancer tissue samples served as the experimental groups for tumor detection.
2. DNA extraction
DNA was separately extracted from the experimental group and the control group. In this experiment, the Epiprobe Biotech Genomic DNA Extraction Kit (Epiprobe Biotech, K-21) was used for DNA extraction (but the present disclosure is not limited to this method).
3. Bisulfite Treatment
The extracted DNA was modified using bisulfite. In this experiment, the EZ DNA Methylation-Gold Kit from ZYMO Research (catalog no. D5006) was used, strictly following the steps in the instruction manual (but the present disclosure is not limited to this kit).
4. PCR Amplification and Library Construction
After modification, the target region was enriched by PCR amplification using primers designed for the bisulfite-treated target region. The primer sequences are shown in Table 6.

**Table 6**

| **Target** | **Primer** | **Sequences (5'-3')** |
|---|---|---|
| TAGMe-1 | Forward primer | ttatttaggttattgttagttgattttaaa (SEQ ID NO: 13) |
| | Reverse primer | ctttaacctaacrcacacrcctctaaaacctaa (SEQ ID NO: 14) |
| TAGMe-2 | Forward primer | ttgtagggaayggttttagtataggtag (SEQ ID NO: 15) |
| | Reverse primer | tacccttcccctcctctcctaaataacctaa (SEQ ID NO: 16) |

Subsequently, the amplified products were purified, and sequencing adapters were ligated to construct the NGS sequencing library.

### 5. NGS sequencing

Detection was performed using Illumina's HiSeq X high-throughput sequencer, strictly following the steps in the instruction manual (but the present disclosure is not limited to this model of high-throughput sequencer).

### 6. Calculation of methylation values for TAGMe-1 and TAGMe-2

The target NGS sequencing data comprise the methylation status of every individual CpG site within the target region. The methylation level of TAGMe-1 and TAGMe-2 is calculated as follows: the methylation level at an individual site = (number of methylated reads at that site)/(total number of reads); the methylation level of the target region = the average of the methylation levels of all sites.

### 7. Analysis results of single marker

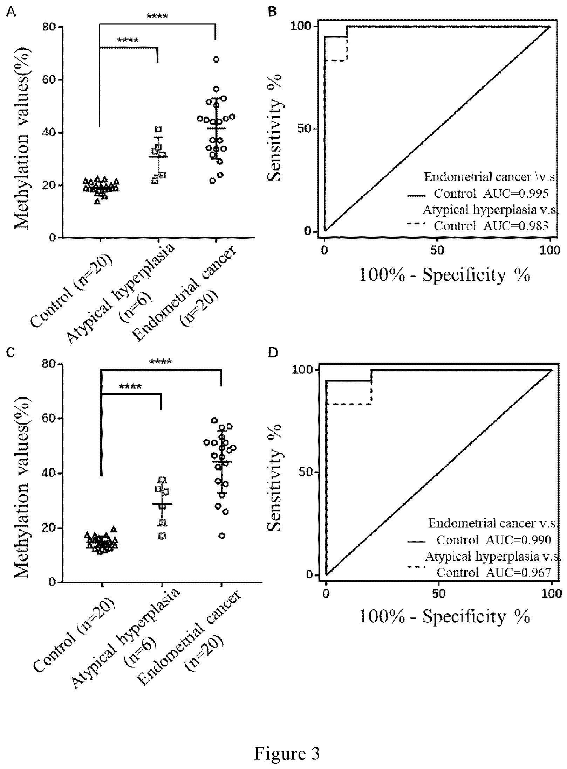

The target NGS results for the tissues are shown in Figure 3. The methylation level differences of TAGMe-1 and TAGMe-2 between the paracancerous/normal control group and the tumor experimental group were compared, and ROC analysis was performed. For TAGMe-1, as shown in Figure 3A, in clinical samples of endometrial cancer, the methylation values in the atypical hyperplasia and cancer tissue samples were significantly higher than those in the control group. The ROC analysis for TAGMe-1 is shown in Figure 3B, with an AUC of 0.995 for distinguishing the endometrial cancer group from the control group and an AUC of 0.983 for distinguishing the atypical hyperplasia group from the control group, both exhibiting excellent discrimination performance. When the threshold is set at the maximum Youden index, the sensitivity and specificity of TAGMe-1 in distinguishing the endometrial cancer group from the control group are 95% and 100%, respectively; and for distinguishing atypical hyperplasia from the control group, the sensitivity and specificity are 83.33% and 90%, respectively.

For TAGMe-2, as shown in Figure 3C, in clinical samples of endometrial cancer, the methylation values in the atypical hyperplasia and cancer tissue samples were significantly higher than those in the control group. The ROC analysis for TAGMe-2 is shown in Figure 3D, with an AUC of 0.990 for distinguishing the endometrial cancer group from the control group and an AUC of 0.967 for distinguishing the atypical hyperplasia group from the control group, both exhibiting excellent discrimination performance. When the threshold is set at the maximum Youden index, the sensitivity and specificity of TAGMe-2 in distinguishing the endometrial cancer group from the control group are 95% and 100%, respectively; and for distinguishing atypical hyperplasia from the control group, the sensitivity and specificity are 83.33% and 100%, respectively.

### 8. Analysis of a combination of markers

A combination of TAGMe-1 and TAGMe-2 was detected, and their combined performance is shown in Table 7.

**Table 7. Analysis of a combination of TAGMe-1 and TAGMe-2**

| **Indicators for detection** | **TAGMe-1 for detecting atypical endometrial hyperplasia** | **TAGMe-1 for detecting endometrial cancer** | **TAGMe-2 for detecting atypical endometrial hyperplasia** | **TAGMe-2 for detecting endometrial cancer** | **Combination of TAGMe-1 and TAGMe-2 for detecting atypical endometrial hyperplasia** | **Combination of TAGMe-1 and TAGMe-2 for detecting endometrial cancer** |
|---|---|---|---|---|---|---|
| **Sensitivity** | 83.33% | 95.00% | 83.33% | 95.00% | 95.00% | 100.00% |
| **Specificity** | 90.00% | 100.00% | 100.00% | 100.00% | 100.00% | 100.00% |
| Total consistency rate | 88.46% | 97.50% | 96.15% | 97.50% | 98.00% | 100.00% |
| Positive predictive value | 71.43% | 100.00% | 100.00% | 100.00% | 100.00% | 100.00% |
| Negative predictive value | 94.74% | 95.24% | 95.24% | 95.24% | 98.00% | 100.00% |

The results show that the combination of the two has a synergistic effect and further increases the sensitivity of methylation markers in detecting endometrial cancer and precancerous lesions.

### Example 4: DNA methylation detection in endometrial cancer-paracancerous tissue samples: pyrosequencing method

In this example, DNA methylation analysis in endometrial cancer-paracancerous tissue samples for marker analysis is performed using the pyrosequencing method. The steps are as follows:
1. Acquisition of clinical samples: 25 cases of normal cervical exfoliated cell samples, 5 cases of cervical exfoliated cell samples from patients with endometrial atypical hyperplasia, and 15 cases of cervical exfoliated cell samples from patients with endometrial cancer were obtained clinically. The normal cervical exfoliated cell samples served as the control group, and the cervical exfoliated cell samples from patients with atypical hyperplasia and endometrial cancer served as the experimental group for tumor detection.
2. DNA extraction: DNA was extracted from the clinical samples. In this experiment, the Epiprobe Biotech Genomic DNA Extraction Kit (catalog no. K-21) was used (but the present disclosure is not limited to this method).
3. Bisulfite treatment
   The extracted DNA was modified using bisulfite. In this experiment, the EZ DNA Methylation-Gold Kit from ZYMO Research (catalog no. D5006) was used, strictly following the steps in the instruction manual (but the present disclosure is not limited to this method).
4. Primer design
   Based on the TAGMe-1 and TAGMe-2 sequences (SEQ ID NO: 1 and SEQ ID NO: 5), PCR amplification primers and pyrosequencing primers were designed respectively to detect the methylation values at the corresponding CpG sites on the target sequence, serving as representatives of their methylation levels. The PCR amplification primer sequences, the pyrosequencing primer sequences, the pyrosequencing read sequences and the detection sites are the same as those in Example 1.
5. PCR amplification and agarose gel electrophoresis
   Using the bisulfite-treated samples as the PCR template, PCR amplification was performed. The specificity of the PCR amplification was verified by agarose gel electrophoresis of the amplified products.
6. Pyrosequencing
   Detection was carried out using the PyroMark Q96 ID pyrosequencing instrument from QIAGEN, strictly following the steps provided in the instruction manual.
7. Methylation value calculation
   The methylation status of each individual CpG site within the target region can be detected by pyrosequencing independently. The average methylation value of all CpG sites is calculated as the methylation value of TAGMe-1 and TAGMe-2 in the sample.
8. Result analysis

The methylation level differences of TAGMe-1 and TAGMe-2 between the paracancerous/normal control group and the tumor experimental group were compared, and ROC analysis was performed.

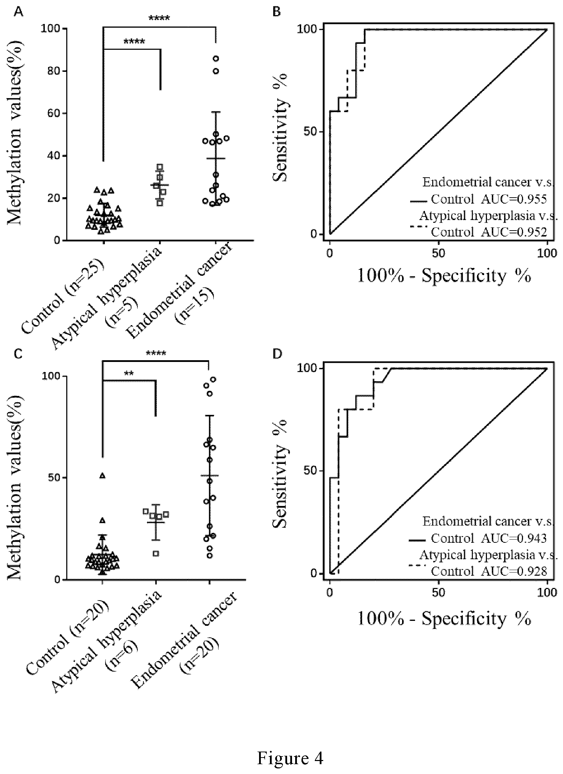

The analysis result of TAGMe-1 is shown in Figure 4A. In clinical samples of endometrial cancer, the methylation values of TAGMe-1 in the atypical hyperplasia and cancer tissue samples were significantly higher than those in the control group. The ROC analysis for TAGMe-1 is shown in Figure 4B, with an AUC of 0.955 for distinguishing the endometrial cancer group from the control group and an AUC of 0.952 for distinguishing the atypical hyperplasia group from the control group, both exhibiting excellent discrimination performance. When the threshold is set at the maximum Youden index, the sensitivity and specificity of TAGMe-1 in distinguishing the endometrial cancer group from the control group are 100% and 84%, respectively; and for distinguishing atypical hyperplasia from the control group, the sensitivity and specificity are 100% and 84%, respectively.

The analysis result of TAGMe-2 is shown in Figure 4C. In clinical samples of endometrial cancer, the methylation values of TAGMe-2 in the atypical hyperplasia and cancer tissue samples were significantly higher than those in the control group. The ROC analysis for TAGMe-2 is shown in Figure 4D, with an AUC of 0.943 for distinguishing the endometrial cancer group from the control group and an AUC of 0.928 for distinguishing the atypical hyperplasia group from the control group, both exhibiting excellent discrimination performance. When the threshold is set at the maximum Youden index, the sensitivity and specificity of TAGMe-2 in distinguishing the endometrial cancer group from the control group are 86.67% and 88%, respectively; and for distinguishing atypical hyperplasia from the control group, the sensitivity and specificity are 100% and 80%, respectively.

### 9. Analysis of a combination of TAGMe-1 and TAGMe-2

A combination of TAGMe-1 and TAGMe-2 was detected, and their combined performance in detecting endometrial cancer and precancerous lesions was further analyzed, as shown in Table 8.

**Table 8. Analysis of a combination of TAGMe-1 and TAGMe-2**

| Detection indicatiors | TAGMe-1 for detecting atypical endometrial hyperplasia | TAGMe-1 for detecting endometrial cancer | TAGMe-2 for detecting atypical endometrial hyperplasia | TAGMe-2 for detecting endometrial cancer | Combination of TAGMe-1 and TAGMe-2 for detecting atypical endometrial hyperplasia | Combination of TAGMe-1 and TAGMe-2 for detecting endometrial cancer |
|---|---|---|---|---|---|---|
| Sensitivity | 100.00% | 100.00% | 100.00% | 86.67% | 100.00% | 100.00% |
| Specificity | 84.00% | 84.00% | 80.00% | 88.00% | 96.00% | 92.00% |
| Total consistency rate | 86.67% | 90.00% | 83.33% | 87.50% | 96.67% | 95.00% |
| Positive predictive value | 55.56% | 78.95% | 50.00% | 81.25% | 83.33% | 91.43% |
| Negative predictive value | 100.00% | 100.00% | 100.00% | 91.67% | 100.00% | 100.00% |

It is noted that paracancerous tissue samples cannot simply be regarded as normal samples because, during clinical sampling, paracancerous tissues may be contaminated with some cancer tissue or may undergo certain epigenetic variations due to the influence of the cancerous lesion, leading to a decrease in specificity. Therefore, the specificity in some samples in the table may fluctuate compared to the previous examples. Nevertheless, the combined detection of the two markers can significantly improve the specificity, overall concordance, and positive predictive value in detecting endometrial atypical hyperplasia.

### Example 5. DNA methylation detection in endometrial cancer-cervical exfoliated cell samples

In this example, DNA methylation analysis in endometrial cancer-cervical exfoliated cell samples for marker analysis is performed using the Me-qPCR method. The steps are as follows:
1. Acquisition of clinical samples: 38 cases of normal cervical exfoliated cell samples, 8 cases of cervical exfoliated cell samples from patients with endometrial atypical hyperplasia, and 33 cases of cervical exfoliated cell samples from patients with endometrial cancer were obtained clinically. The normal cervical exfoliated cell samples served as the control group, and the cervical exfoliated cell samples from patients with atypical hyperplasia and endometrial cancer served as the experimental group for tumor detection.
2. DNA extraction: DNA was extracted from the clinical samples. In this experiment, the Epiprobe Biotech Genomic DNA Extraction Kit ( K-21) was used (but the present disclosure is not limited to this method).
3. Enzymatic digestion reaction: The DNA was digested using a methylation-sensitive restriction enzyme. Unmethylated digestion sites will be cleaved. In this experiment, the HpaII enzyme (NEB, R0171) was used for the enzymatic digestion reaction (but the present disclosure is not limited to this method).
4. Primer design: Based on the TAGMe-1 and TAGMe-2 sequences, corresponding amplification primers were designed to amplify the products resulting from the methylation-sensitive restriction enzyme digestion. The abundance of the digested DNA fragments is detected and used to represent the methylation values of TAGMe-1 and TAGMe-2, with the GAPDH gene serving as an internal control.
5. qPCR amplification: Using the enzyme-digested samples as the qPCR template, qPCR amplification was performed. In this experiment, the ABI 7500 qPCR instrument from Thermo was used (but the present disclosure is not limited to this method).
6. Calculation of Methylation Values: The DNA methylation level of each sample was evaluated using the following formula:ΔCt_{_the marker of the present disclosure} = Ct_{_the marker of the present disclosure} - Ct_{_GAPDH}. A smaller ΔCt indicates a higher methylation level.
7. Result analysis: The differences in the methylation values of TAGMe-1 and TAGMe-2 between the control group and the tumor experimental group were compared, and ROC analysis was performed.

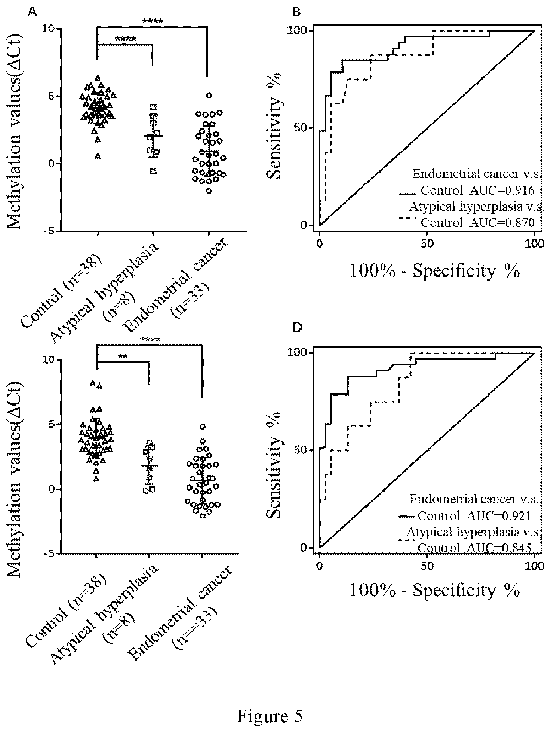

Me-qPCR results of cervical cells are shown in Figure 5. The result of TAGMe-1 is shown in Figure 5A. In clinical samples of endometrial cancer, the methylation values of TAGMe-1 in the atypical hyperplasia and cancer tissue samples were significantly higher than those in the control group. The ROC analysis for TAGMe-1 is shown in Figure 5B, with an AUC of 0.916 for distinguishing the endometrial cancer group from the control group and an AUC of 0.870 for distinguishing the atypical hyperplasia group from the control group, both exhibiting excellent discrimination performance. When the threshold is set at the maximum Youden index, the sensitivity and specificity of TAGMe-1 in distinguishing the endometrial cancer group from the control group are 84.85% (68.1%-94.89%) and 89.47% (75.2%-97.06%), respectively; and for distinguishing atypical hyperplasia from the control group, the sensitivity and specificity are 87.5% (47.35%-99.68%) and 76.32% (59.76%-88.56%), respectively. The result of TAGMe-2 is shown in Figure 5C. In clinical samples of endometrial cancer, the methylation values of TAGMe-2 in the atypical hyperplasia and cancer tissue samples were significantly higher than those in the control group. The ROC analysis for TAGMe-2 is shown in Figure 5D, with an AUC of 0.921 for distinguishing the endometrial cancer group from the control group and an AUC of 0.845 for distinguishing the atypical hyperplasia group from the control group, both exhibiting excellent discrimination performance. When the threshold is set at the maximum Youden index, the sensitivity and specificity of TAGMe-2 in distinguishing the endometrial cancer group from the control group are 87.88% and 86.84%, respectively; and for distinguishing atypical hyperplasia from the control group, the sensitivity and specificity are 100% and 57.89%, respectively.

8. Analysis of a combination of TAGMe-1 and TAGMe-2: A combination of TAGMe-1 and TAGMe-2 was detected, and their combined performance in detecting endometrial cancer and precancerous lesions was further analyzed, as shown in Table 8.

**Table 9. Analysis of a combination of TAGMe-1 and TAGMe-2**

| Indicators for detection | TAGMe-1 for detecting atypical endometrial hyperplasia | TAGMe-1 for detecting endometri al cancer | TAGMe-2 for detecting atypical endometrial hyperplasia | TAGMe-2 for detecting endometri al cancer | Combination of TAGMe-1 and TAGMe-2 for detecting atypical endometrial hyperplasia | Combination of TAGMe-1 and TAGMe-2 for detecting endometrial cancer |
|---|---|---|---|---|---|---|
| Sensitivity | 87.50% | 84.85% | 100.00% | 87.88% | 100.00% | 93.94% |
| Specificity | 76.32% | 89.47% | 57.89% | 86.84% | 78.95% | 92.13% |
| Total consistency rate | 78.26% | 87.32% | 65.22% | 87.32% | 80.43% | 88.73% |
| Positive predictive value | 43.75% | 87.50% | 33.33% | 85.29% | 46.67% | 93.82% |
| Negative predictive value | 96.67% | 87.18% | 100.00% | 89.19% | 100.00% | 94.12% |

According to the above, the combined detection of the two markers can significantly improve the total consistency rate in detecting endometrial atypical hyperplasia, as well as the sensitivity and consistency rate in detecting endometrial cancer.

### Example 6. DNA methylation detection in endometrial cancer-cervical exfoliated cell samples (the Me-qPCR method)

19 cases of normal cervical exfoliated cell samples, 6 cases of cervical exfoliated cell samples from patients with endometrial atypical hyperplasia, and 21 cases of cervical exfoliated cell samples from patients with endometrial cancer were obtained clinically. The normal cervical exfoliated cell samples served as the control group, and the cervical exfoliated cell samples from patients with atypical hyperplasia and endometrial cancer served as the experimental group for tumor detection. Following the steps of Me-qPCR detection described in Example 5, the differences in the methylation values of TAGMe-1 and TAGMe-2 between the control group and the tumor experimental group were compared and ROC analysis was performed.

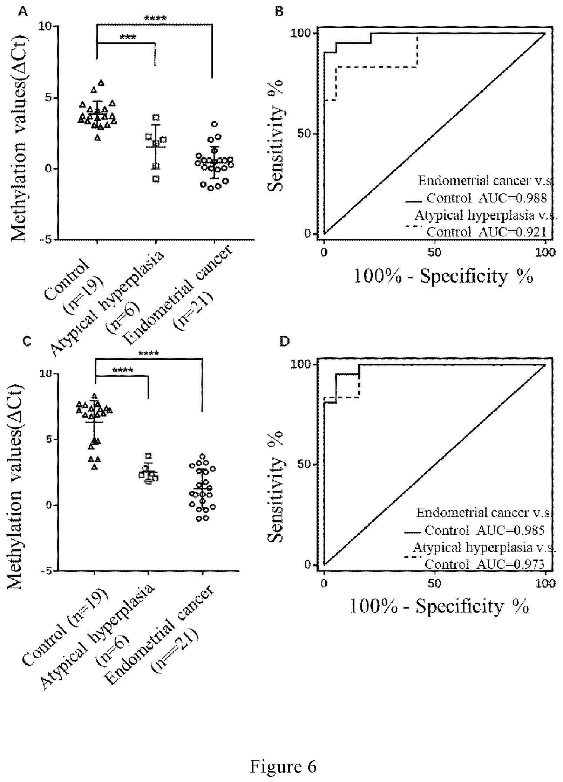

Me-qPCR results of cervical cells are shown in Figure 6. The result of TAGMe-1 is shown in Figure 6A. In clinical samples of endometrial cancer, the methylation values of TAGMe-1 in the atypical hyperplasia and cancer tissue samples were significantly higher than those in the control group. The ROC analysis is shown in Figure 6B, with an AUC of 0.988 for TAGMe-1 distinguishing the endometrial cancer group from the control group and an AUC of 0.921 for TAGMe-1 distinguishing the atypical hyperplasia group from the control group, both exhibiting excellent discrimination performance. When the threshold is set at the maximum Youden index, the sensitivity and specificity of TAGMe-1 in distinguishing the endometrial cancer group from the control group are 90.48% and 100%, respectively; and for distinguishing atypical hyperplasia from the control group, the sensitivity and specificity are 83.33% and 94.74%, respectively.

The result of TAGMe-2 is shown in Figure 6C. In clinical samples of endometrial cancer, the methylation values of TAGMe-2 in the atypical hyperplasia and cancer tissue samples were significantly higher than those in the control group. The ROC analysis is shown in Figure 6D, with an AUC of 0.985 for TAGMe-2 distinguishing the endometrial cancer group from the control group and an AUC of 0.973 for TAGMe-2 distinguishing the atypical hyperplasia group from the control group, both exhibiting excellent discrimination performance. When the threshold is set at the maximum Youden index, the sensitivity and specificity of TAGMe-2 in distinguishing the endometrial cancer group from the control group are 95.24% and 94.74%, respectively; and for distinguishing atypical hyperplasia from the control group, the sensitivity and specificity are 100% and 84.21%, respectively.

TAGMe-1 and TAGMe-2 were detected in combination to further analyze the sensitivity of the methylation markers for detecting endometrial cancer and precancerous lesions. The combined performance is shown in Table 10.

**Table 10. Analysis of a combination of TAGMe-1 and TAGMe-2**

| Detection indicatiors | TAGMe-1 for detecting atypical endometrial hyperplasia | TAGMe-1 for detecting endometrial cancer | TAGMe-2 for detecting atypical endometrial hyperplasia | TAGMe-2 for detecting endometri al cancer | Combination of TAGMe-1 and TAGMe-2 for detecting atypical endometrial hyperplasia | Combination of TAGMe-1 and TAGMe-2 for detecting endometrial cancer |
|---|---|---|---|---|---|---|
| Sensitivity | 83.33% | 90.48% | 100.00% | 95.24% | 100.00% | 100.00% |
| Specificity | 94.74% | 100.00% | 84.21% | 94.74% | 100.00% | 100.00% |
| Total consistency rate | 92.00% | 95.00% | 88.00% | 95.00% | 100.00% | 100.00% |
| Positive predictive value | 83.33% | 100.00% | 66.67% | 95.24% | 100.00% | 100.00% |
| Negative predictive value | 94.74% | 90.48% | 100.00% | 94.74% | 100.00% | 100.00% |

According to the above, the combined detection of the two markers can evidently improve the total consistency rate in detecting endometrial atypical hyperplasia.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A use of an isolated nucleic acid or a nucleic acid converted therefrom in preparing reagents or kits for tumor detection;
wherein, the nucleic acid is: (1) a nucleic acid with a nucleotide sequence shown in SEQ ID NO: 1 or SEQ ID NO: 5, or a nucleic acid combination thereof; or (2) a nucleic acid with a sequence complementary to the nucleic acid of (1), or a nucleic acid combination thereof;
wherein, the nucleic acid converted from the isolated nucleic acid is a nucleic acid corresponding to (1) or (2), with unmodified cytosine converted to T or U and modified cytosine C at CpG sites unchanged.

2. The use according to claim 1, wherein the nucleic acid with a sequence complementary to the nucleic acid of (1) is a nucleic acid with a nucleotide sequence shown in SEQ ID NO: 2 or SEQ ID NO: 6.

3. The use according to claim 1, wherein the tumor comprises: endometrial cancer, cervical cancer, pancreatic cancer, head and neck cancer, colon cancer, colorectal cancer, esophageal cancer, lung cancer, cholangiocarcinoma.

4. The use according to claim 1, wherein the tumor detection is for tumors or precancerous lesions thereof.

5. The use according to claim 1, wherein the tumor detection can be used for samples comprising: blood samples, tissue samples, cervical samples, uterine cavity samples, pleural effusion samples, bronchoalveolar lavage fluid samples, ascites samples, ascites lavage fluid samples, bile samples, fecal samples, urine samples, saliva samples, cerebrospinal fluid samples, cell smear samples, cell samples; preferably, the samples comprises: cervical scraping or brushing samples, cervical swabs, uterine cavity tissue, cervical exfoliated cells, endocervical curettage specimens, uterine lavage fluids, vaginal secretions.

6. A method for detecting the methylation level of a sample to be tested, wherein the method comprises:
extracting nucleic acids from the sample to be tested; and
detecting the modification status of CpG sites in a target sequence or fragment thereof within the extracted nucleic acids, wherein the target sequence is a nucleic acid converted from a nucleotide sequence shown in SEQ ID NO: 1 or SEQ ID NO: 5, or a nucleic acid combination thereof, with unmodified cytosine converted to T or U and modified cytosine C at CpG sites unchanged, corresponding to the nucleotide sequence shown in SEQ ID NO: 1 or SEQ ID NO: 5.

7. The method according to claim 6, wherein the method for detecting the modification status of the CpG sites in the target sequence of extracted nucleic acids comprises pyrosequencing, bisulfite conversion sequencing, methylation microarray, methylation-specific PCR, methylation-sensitive restriction enzyme digestion, qPCR, digital PCR, next-generation sequencing, third-generation sequencing, whole-genome methylation sequencing, DNA enrichment assay, simplified bisulfite sequencing, HPLC, MassArray, or combinations thereof.

8. The method according to claim 6, wherein the method for detecting the modification status of the CpG sites in the target sequence of extracted nucleic acids comprises: (i) treating the extracted nucleic acids to convert unmodified cytosine to uracil; preferably, the modification comprises 5-methylation, 5-hydroxymethylation, 5-formylmethylation, or 5-carboxymethylation.

9. The method according to claim 8, wherein it further comprises: (i) subjecting the nucleic acids to bisulfite treatment as described in step (i); and (ii) analyzing the modification status of the target sequence in the nucleic acids treated in step (i).

10. A method for preparing a reagent for tumor detection, wherein the method comprises:
providing a nucleic acid with a nucleotide sequence shown in SEQ ID NO: 1 or SEQ ID NO: 5, wherein the full-length nucleic acid or a fragment thereof is used as a target sequence, and designing a detection reagent for specifically detecting modifications at CpG sites of the target sequence.

11. The method according to claim 10, wherein the detection reagent comprises but is not limited to: primers, probes, chips or test strips.

12. A reagent or reagent combination for specifically detecting modifications at CpG sites in a target sequence, wherein the target sequence is a nucleic acid converted from a nucleotide sequence shown in SEQ ID NO: 1 or SEQ ID NO: 5, or a reagent combination thereof; preferably, the reagent or reagent combination targets a gene sequence comprising the target sequence; preferably, the gene sequence comprises gene Panel or gene cluster; preferably, the reagent or reagent combination is selected from:
primers with the sequences of SEQ ID NO: 9 and SEQ ID NO: 10;
primers with the sequences of SEQ ID NO: 11 and SEQ ID NO: 12;
primers with the sequences of SEQ ID NO: 13 and SEQ ID NO: 14; or
primers with the sequences of SEQ ID NO: 15 and SEQ ID NO: 16.

13. A use of the reagent or reagent combination according to claim 12 for preparing kits for tumor detection; preferably the tumor comprises: endometrial cancer, cervical cancer, pancreatic cancer, head and neck cancer, colon cancer, colorectal cancer, esophageal cancer, lung cancer, cholangiocarcinoma.

14. A kit for tumor detection, wherein the kit comprises the reagent or reagent combination according to claim 12.

15. An isolated nucleic acid or nucleic acid converted therefrom, wherein the nucleic acid is:
(1) a nucleic acid with a nucleotide sequence shown in SEQ ID NO: 1 or SEQ ID NO: 5, or a nucleic acid combination thereof; or
(2) a nucleic acid with a sequence complementary to the nucleic acid of (1), or a nucleic acid combination thereof;
wherein, the nucleic acid converted from the isolated nucleic acid is a nucleic acid corresponding to (1) or (2), with unmodified cytosine converted to T or U and modified cytosine C at CpG sites unchanged.
